# EUROPEAN PATENT SPECIFICATION

(11) **EP 0 991 382 B1**
(45) Date of publication and mention of the grant of the patent: **28.04.2004**
(21) Application number: 98924070.0
(22) Date of filing: 03.06.1998
(51) Int. Cl.: A61F 5/443

(54) **AN OSTOMY APPLIANCE**
OSTOMIEVORRICHTUNG
ACCESSOIRE DE STOMIE

(30) Priority: 04.06.1997 DK 65897
(43) Date of publication of application: 12.04.2000
(73) Proprietor: COLOPLAST A/S, 3050 Humlebaek (DK)
(72) Inventor: NIELSEN, Inger, Mann, DK-2000 Frederiksberg (DK); OLSEN, Eskil, Hojland, DK-2930 Klampenborg (DK); GOTHJAELPSEN, Laila, Busk, DK-2650 Hvidovre (DK)
(74) Representative: Olsen, Lars Pallisgaard
(86) International application number: PCT/DK1998/000235
(87) International publication number: WO 1998/055057

(56) References cited:
- DK-L- 23 091
- GB-A- 1 587 604
- GB-A- 2 041 753
- GB-A- 2 083 762
- US-A- 3 898 990
- US-A- 4 258 715

## Description

The present invention relates to an ostomy appliance.

### BACKGROUND OF THE INVENTION

In connection with surgery for a number of diseases in the gastro-intestinal tract a consequence is, in many cases, that the patient is left with an abdominal stoma such as a colostomy, an ileostomy or a urostomy. In such cases or in connection with a fistula the patient will have to rely on an external ostomy appliance to collect the bedily waste material emerging from such opening.

Ostomy appliances are well known. Such appliances may be two-piece or one-piece appliances. In both types of appliances, a collection bag is attached to the user's abdomen in the region of the stoma for receiving waste material or exudates from the ostomy. With two-piece appliances an adhesive faceplate or body side member is first attached to the user's abdomen, and a receiving member or bag is then attached to the body side ostomy member for receiving waste material from the ostomy. In one-piece appliances a collection bag has an integrated adhesive faceplate for attaching the bag to the user.

Such a one-piece appliance is e.g. described in GB Patent publication GB-A-1 587 604 which discloses an ostomy appliance in the form of a one-piece drainage pouch which may be attached to a users body by use of an adhesive. The drainage pouch comprises an integrated deformable sealing ring or pad.

When using one-piece appliances, the whole appliance, including the adhesive wafer or pad securing the appliance to the skin is removed and replaced by a fresh appliance. When using two-piece appliances, the body side ostomy member is often left in place for several days, and only the receiving member or bag is replaced.

The service time of the body side ostomy member depends on the amount and the aggressiveness of the exudates and of the tightness of the sealing between the ostomy and the body side ostomy member.

In the known appliances it is necessary to change the body side member of two-piece appliance when the centre part of the adhesive wafer or pad has been sufficiently deteriorated to allow access of the aggressive exudates to the skin surrounding the stoma, irrespective of the fact that the wafer as such has a much longer wearing time. The access of aggressive exudates to the skin is causing skin problems.

Skin problems are common for persons having a stoma. Generally, about 40 % of them have skin problems (Pearl et al. 1985 "Early local complications from intestinal stomas", Arch. Surg. 120; 1145-1147.) and the frequency is especially high for persons having an urostomy or ileostomy. About 80 % of the persons having an ileostomy have skin problems (Hellman, J.D., Lago, C.P. 1990 "Dermatologic complications in colostomy and ileostomy patients", International Journal of Dermatology, 29 (2); 129-133.). The skin problems are mostly pronounced in a circular area about the stoma (10-15 mm from the stoma) (Hellman and Lago 1990).

Frequent changing of the body side member of a two-piece appliance or the frequent exchange of a one-piece appliance is undesirable due to the irritation of the skin, and if the intervals between exchanging of the body side member can be increased, then the nuisance of wearing an ostomy appliance can be reduced and the user's quality of life may consequently be improved.

It is known to place a ring with a convex proximal side on the skin before applying the body side member or to make a filling between the edge of the stoma and the shaped opening of the ostomy appliance in order to form a seal between the stoma and the ostomy appliance in order to alleviate the problems using a commercially available medical grade adhesive paste. Such pastes are e.g. sold by Bristol-Myers Squibb under the trademark Stomahesive® or by Coloplast under the trade mark Coloplast® Paste.

These pastes or sealing compounds, however, do not have a composition which has a sufficiently cohesion ensuring safe removal thereof without leaving residues on the skin and, on the other hand, the pastes often are so sticky that they cannot easily be shaped using the finger without sticking to the finger.

A paste should preferably have a composition which is sufficiently tacky to secure the appliance or skin barrier to the skin of the user's abdomen, a cohesion of the paste itself ensuring safe removal thereof without leaving residues on the skin. On the other hand, the paste must not be so sticky that it cannot easily be shaped by a finger or hand without sticking to the hand. Furthermore, the paste must show a sufficient elasticity in order to be able to follow the movements of the patient without slipping the skin and should also show a great resistance to erosion caused by aggressive exudates from an ostomy.

In GB Patent Application No. GB 2 290 974 is disclosed an ostomy appliance wherein a body-side is combined with a mouldable substance of non-hypoallergenic, non-memory putty-like adhesive, particularly based on hydrocolloid or hydrogel.

GB Patent Application No. GB 2 290 974 discloses a body-side ostomy member comprising a ring to which a bag-side coupling ring or a bag can be attached, said ring comprising a rib and a flange, said flange being mounted on a wafer of medical grade adhesive having a central opening of diameter at least 65 % of the internal diameter of the ring. A mouldable substance of non-hypoallergenic, non-memory putty-like adhesive, particularly based on hydrocolloid or hydrogel, is disposed radially inward of the wafer so that it forms a protective substance surrounding the stoma. The mouldable substance has a thickness of 1.25 - about 3 times that of the wafer and a central opening therein of a diameter no more than 1/10 of the internal diameter of the ring. Both the medical grade adhesive and the mouldable adhesive are adhered to the skin.

European Patent application No. EP 0 686 381 discloses an ostomy appliance comprising a collection pouch and faceplate assembly including a flexible patch having a stoma-receiving opening, a first layer of skin friendly hydrocolloid-containing adhesive material along one side of said patch about said opening for securing said faceplate assembly to peristomal skin surfaces, and a second layer of relatively soft, easy-deformable and extrudable, adhesive sealant material of a composition that is resistant to being dissolved or disintegrated by stomal fluids and that immediately surrounds said opening; said second layer being displaceable inwardly and axially into said opening for forming a stoma-engaging annular gasket to prevent stomal fluids from contacting the peristomal skin and said first adhesive layer.

The mouldable substance of non-hypoallergenic, non-memory putty-like adhesive or flexible patch disclosed in GB Patent Application No. GB 2 290 974 and European Patent application No. EP 0 686 381 both are secured to the rim of the opening for receiving the stoma.

The ostomy appliances disclosed in GB Patent Application No. GB 2 290 974 and European Patent application No. EP 0 686 381 both suffer from the drawback that the mouldable sealing material is only foreseen to be changed together with the body side member of the appliance, which typically left on the user's skin for several days, whereas the collection bag is changed more frequently, typically several times each day. There is no teaching nor indication that the mouldable sealing material might be exchanged together with the collection bag or even be an integral part thereof.

With an ostomy appliance in accordance with the invention is defined in claim 1, wherein the mouldable adhesive substance is applied to the skin of the user and exchanged together with the collection bag. The mouldable adhesive substance ensures a perfectly close or tight fit to the stoma, which is desirable. The fitting of the mouldable adhesive substance to the stoma can be done either prior to attaching the fresh collection bag to the user's skin, or immediately thereafter, and the user can mould the mouldable adhesive substance with his fingers from the outside of the collection bag, whereby the outer sheet of the bag is situated between the user's fingers and the mouldable adhesive substance. The user will thus not have to touch the mouldable adhesive substance directly with his fingers, and the mouldable adhesive substance is moulded *in situ* which gives a perfect fit to the stoma.

### BRIEF DESCRIPTION OF THE DRAWINGS

The invention is disclosed more in detail with reference to the drawings in which
Fig.1 shows a sectional view of a two-piece ostomy appliance,
Fig.2 shows a first variant of the body side member of the ostomy appliance in fig. 1, and
Fig. 3 shows a second variant of the body side member of the ostomy appliance in fig. 1.

All drawings are schematic drawings that are not necessarily drawn to scale but are merely intended to illustrate the principles of the invention.

### DETAILED DESCRIPTION OF THE INVENTION

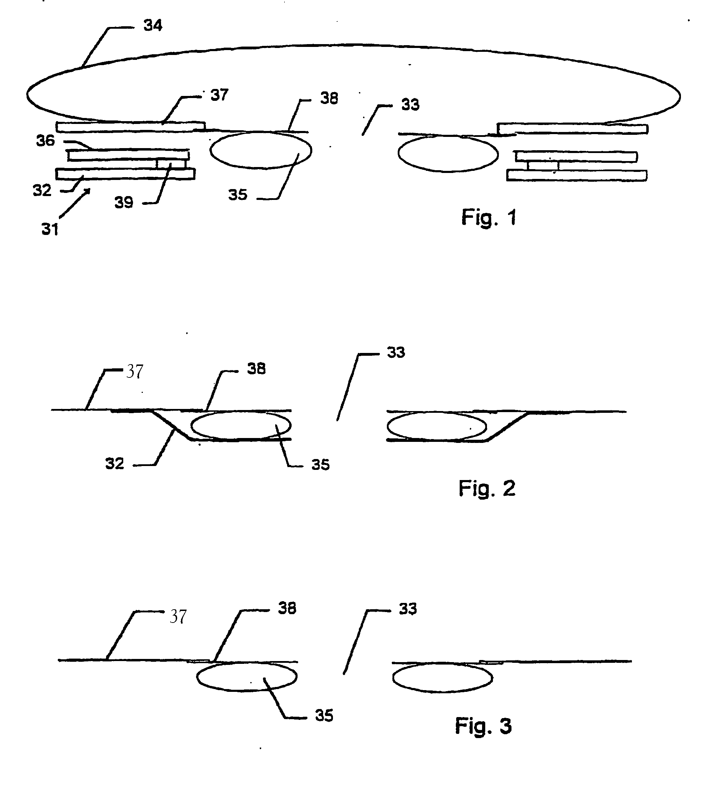

Fig. 1 shows a two-piece ostomy appliance, however, the principles of the shown embodiment apply equally well to one-piece ostomy appliances. The two-piece ostomy appliance in fig. 1 comprises a body side member 31 having a central opening 33 and an adhesive wafer or pad 32 of a skin friendly, medical grade adhesive, also known as a skin barrier. The body side member 31 is intended for securing to a user's skin (not shown) by means of the adhesive pad 32 such that the central opening 33 receives the user's stoma. The body side member 31 has a flange 36 for mounting a receiving member such as a collection bag for collecting bodily excretions or exudates from the user's stoma. The adhesive pad 32 preferably has a (not shown) stabilising backing layer of a flexible material, and the adhesive pad 32 is secured to the flange 36 by means of an adhesive 39 or a welding or other suitable method.

A receiving member or collection bag 34 can be secured to the body side member 31, for which purpose the collection bag has a flange 37. When mounting the collection bag 34 on the body side member 31, the flange 37 of the collection bag will be secured to the flange 36, and for this purpose one (or both) of the contacting surfaces of the two flanges has a layer of adhesive (not shown). In an alternative embodiment the collection bag 34 can be secured to the body side member 31 by means of mechanical coupling means having mating parts on the two flanges 36 and 37. Such mechanical coupling means are known in the art.

The ostomy device in fig. 1 furthermore has a ring shaped, adhesive sealing member or substance 35 around the opening 33. It should be observed that the device alternatively could have had the sealing substance at a part of the edge of the opening only, e.g. the lower part. The sealing member 35 is preferably a uniform, plastically mouldable substance of a hypoallergenic, substantially non-memory putty-like adhesive or it may comprise further constituents such as a protecting film or a mouldable mesh. The ring shaped sealing member 35 is made from a mouldable adhesive substance in the form of a paste of a skin-friendly adhesive being sufficiently tacky to secure the appliance to the abdomen of the user and a cohesion ensuring safe removal thereof without leaving residues on the skin. The ring shaped sealing member 35 is situated on a protecting film or sheet 38 of a soft and mouldable material secured to the flange 36.

In the embodiment in fig. 1 the substance forming the sealing member 35 has properties which make it suitable for use as adhesive for attaching the ostomy device to the peristomal skin of the user, together with the adhesive pad 32. The sealing member 35 is supplied as a ring around the opening 33 and can be moulded or shaped by the user to fit his stoma. The sealing member 35 can be supplied pre-shaped and the final shaping or moulding can be performed by the user either before applying the device to the peristomal skin or immediately thereafter whereby the user will mould or shape the substance of the sealing member from the outside of the collecting bag 34 using his fingers.

Fig.2 shows a body side ostomy member with the essential parts in an alternative embodiment of the invention, where the ring shaped sealing member 35 is softer than in the embodiment in fig. 1. The skin barrier or adhesive pad 32 with its backing layer therefore covers the soft, mouldable sealirg member 35, so that the sealing substance 35 is confited between the protecting film or sheet 38 and the backing layer of the adhesive pad 32. Since the sealing substance 35' in this embodiment is softer than the corresponding substance in fig. 1, this embodiment is more suitable for shaping or moulding the sealing substance after the ostomy appliance has been attached to the peristomal skin of the user by use of releasable mechanical coupling members or releasable adhesive. The shaping or moulding of the sealing substance is performed by the user who uses his fingers and presses or manipulates the sealing substance from the outside of the collection bag, and the sealing substance is thereby squeezed out from between the protecting film or sheet 38 and the backing layer of the adhesive pad 32, and the sealing substance will then form a tight fit to the stoma.

The medical grade adhesive 32 secures the sealing member to the peristomal skin. A variety of such barrier adhesives are known in the art and may be used here, one such formulation being disclosed, for example in the patents DK 147 035 and US 4 551 490. The mouldable adhesive substance 35 is laminated on top of the medical grade adhesive and is used for adapting the sealing member to the stoma by displacing the material inwardly to the stoma by finger pressure. The plastically mouldable adhesive may be composed of a hypoallergenic, soft, easy-deformable, non-memory putty like adhesive material and is preferably a hydrocolloid based adhesive or a hydrogel. The mouldable backing film **38,** e.g. Parafilm® or a polymer solution which is sprayed on the surface, protects the surface of the mouldable substance against dissolution by secrete from the stoma and prevent a tacky surface on the side facing the bag.

The medical grade adhesive improves the performance due to elimination of the risk of dissolution of the mouldable adhesive substance 35. This also eliminates the risk of having residues from the mouldable material remaining on the skin after removal of the appliance. The best performance is achieved if the medical grade adhesive is extended to cover the edges of the mouldable adhesive in order to protect the edges from erosion and dissolution.

Fig. 3 shows a body side ostomy member with the essential parts in an alternative embodiment of the invention, where the ring shaped sealing member 35 is of the same composition as in the embodiment in fig. 1, i.e. less soft than in fig. 2.

The mouldable adhesive substance used in the different compositions of the sealing member is preferably characterised as being a hypoallergenic, substantially plastically mouldable, or non memory putty-like, adhesive. It may e.g. be a homogeneous mixture of a pressure sensitive adhesive component, mineral oil, and hydrocolloid gums or cohesive strengthening agents as the substance disclosed in US patent No. 4 204 540. The substance may also be a composition including one or more hydrocolloids, a film former which is butyl ester of polycarboxylic resin formed from vinyl methyl ether and maleic anhydride, a plasticizer, a thickening agent and an alcohol solvent as disclosed in EP patent No. 0 048 556. A further paste is disclosed in US patent No. 5 369 130. This composition comprises a liquid rubber component and a filler component. The rubber component is a diene-type liquid rubber, preferably butadiene- or isoprene-type. The filler component is selected from the groups consisting of inorganic fillers, natural polymers, semisynthetic water-soluble polymers and synthetic water-soluble polymers. A further composition of a skin protective gel containing polyvinyl methylether or monoisopropyl ester of polyvinylmethylether maleic acid is disclosed in US patent No. 3 876 771. The composition is a made up of a film forming protective colloidal material in combination with a solvent and a gelling agent. Isopropanol is the solvent, monoisopropyl ester of polyvinyl methylether/maleic acid is a film former and polyvinylpyrrolidone, polyvinyl methylether, polyacrylic acid and hydroxypropyl cellulose are the gelling agents. A hydrophilic elastomeric pressure sensitive material is disclosed in US patent No. 4 750 482. This composition is a water-insoluble, hydrophilic, pressure-sensitive adhesive including at least one irradiation cross-linked synthetic organic polymer (predominantly of derived from vinylpyrrolidone) and an adhesive plasticizer (polyethylene glycol).

The composition disclosed in EP 0 048 556 B1 suffers from the drawback that it comprises a considerable amount (25% to 45% by weight) of alcohol, ethanol and isopropanol being preferred. When using such a paste, it should be observed that only a limited time is used for forming the paste after the application, as the paste cures or hardens by evaporation of the alcohol when exposed to air. Furthermore, the amount of alcohol trapped in the paste must be minimised in order to avoid less attractive physical properties due to an adverse effect on the properties of the adhesive of an ostomy appliance which is placed on the paste. Still further, the considerable amount of alcohol may irritate the skin and such a composition is not advisable to use on skin which has been sensibilised.

The pastes disclosed in US patent No. 4 204 540 suffer from the drawback that the shapeability is very dependant of the content of mineral oil. If the composition contains an insufficient amount of mineral oil, then it will be too tough to shape, and if the composition contains too much mineral oil, then the composition becomes sticky and difficult to handle. Generally, pastes based on polyisobutylene, butyl rubber and mineral oil may be very hard, if the content of butyl rubber is high and hence, the paste will be difficult to shape, or it will be very soft and liquid if the content of butyl rubber is low and the content of mineral oil is high.

The substance used in the invention is preferably in the form of a plastically mouldable substance of a hypoallergenic, substantially non-memory putty-like adhesive. In accordance with a preferred embodiment of the invention, the sealing member is made from a mouldable substance of a hypoallergenic, substantially non-memory putty-like adhesive comprising
a) a blockcopolymer having a major content of diblock copolymer,
b) a tackifying liquid constituent, and
c) a waxy constituent.
The sealing member may according to the invention be in the form of a paste a plastically mouldable ring comprising a hypoallergenic, substantially non-memory putty-like adhesive. The substance of the sealing member may have a composition allowing it to cure or harden after being applied to the peristomal skin of the user and after having been shaped or moulded. Such hardening should render the substance soft elastic instead of initially being plastically mouldable or spreadable, whereby the hardened sealing substance will be able to follow possible movements of the skin and the stoma. The hardening process can e.g. be initiated by absorption of water, whereby the composition will form cross links.

The present invention enables the user to apply an ostomy appliance without the use of any tools. Thus, it is only necessary to use body side members having a sufficiently big opening, no adaptation by cutting using scissors is necessary, and the sealing member is adapted snugly to the stoma and the body side member using the fingers.

### MATERIALS AND METHODS OF PRODUCING THE MOULDABLE SEALING SUBSTANCE

Kraton® G1726 from Shell: Styrene-ethylenebutylene-styrene copolymer (SEBS) having a molecular weight of 45,000 as determined by GPC and a content of diblock copolymer of 70%.

Kraton® D1118 from Shell: Styrene-butadiene-styrene copolymer (SBS) having a molecular weight of 103,000 (GPC) and a content of diblock copolymer of 80%.

Vector® 4114 from Exxon: Styrene-isoprene-styrene copolymer (SIS) having a molecular weight of 130,000 and a content of diblock copolymer of 40%

Vistanex® LM-MH from Exxon: polyisobutylene (PIB) having a molecular weight of 90,000 (GPC).

Wax Total 40/60 from TOTAL

Petroleum jelly: Vaselinum Album from Witco

Polybutene oil: Hyvis® 10 from BP having a molecular weight of 1,500.

Polybutene: Hyvis® 2000 from BP having a molecular weight Mw of 30,000

Mineral Oil: PL 500 from Parafluid Mineral Oel

Tackifier resin: Regalite® R91 resin from Hercules or Arkon® P-90 resin from Arakawa

Sodium carboxymethylcellulose: Akucell® AF2881 from Akzo or Blanose® 9H4XF from Hercules Corp.

Guar gum: Guar Gum FG 200 from Nordisk Gelatine

Pectin: Pektin LM 12CG Z from Copenhagen Pectin or Pektin USP/100 from Copenhagen Pectin

Gelatine: Gelatine P.S.98.240.233 from ED. Geistlich Sohne AG

Zinc Oxide: Zinkoxid Pharma from Hoechst AG

A Z mixer Type LKB 025 from Herman-Linden was used.

### EXPERIMENTAL PART

### EXAMPLE 1.

Preparation of a mouldable substance to be used in the invention.

100 grams of Kraton® G1726 was used and the amounts of other ingredients used correspond to the composition stated in Table 1.

Equal amounts of Kraton® G1726 (SEBS) and of Vistanex® LM-MH were mixed in a Z Mixer for 20 minutes at 160 °C under a vacuum of 100 mbar. Then, the vacuum was released, the mixing was continued at 160 °C for 10 minutes and the remains of Vistanex® LM-MH, the wax, and petroleum jelly were admixed and mixed for 10 minutes each. Then, the heating was turned off, and guar gum was added at maximum 90 °C under a vacuum of 100 mbar and mixed for 10 minutes. Finally, pectin, gelatine and zinc oxide were admixed at a temperature of 90 °C and mixed for 10 minutes.

The paste is then ready to use and may preferably be packed in metered amounts, e.g. in a blister pack or rod. A rod may be rolled and have a release liner on one or both sides. The product is preferably produced and packed under aseptic conditions.

### EXAMPLE 2.

Preparation of a mouldable substance to be used in the invention.

100 grams of Kraton® G1726 was used and the amounts of other ingredients used correspond to the composition stated in Table 1.

Equal amounts of Kraton® G1726 (SEBS) and Vistanex® LM-MH were mixed in a Z Mixer for 20 minutes at 160 °C under a vacuum of 100 mbar. Then, the vacuum was released, the mixing was continued at 160 °C for 10 minutes and the remains of Vistanex® LM-MH, the wax, and Hyvis® 10 or PL 500 were admixed and mixed for 10 minutes each. Then, the heating was turned off, and guar gum was added at maximum 90 °C under a vacuum of 100 mbar and mixed for 10 minutes. Finally, pectin, gelatine and zinc oxide were admixed at a temperature of 90 °C and mixed for 10 minutes.

The paste is then ready to use and may preferably be packed in metered amounts, e.g. in a blister pack or rod. A rod may be rolled and have a release liner on one or both sides. The product is preferably produced and packed under aseptic conditions.

### EXAMPLES 3 - 5

Preparation of mouldable substances to be used in the invention.

In the same manner as described in Example 2 above, mouldable substances used in the invention were produced having the compositions stated in the below Table 1:

**Table 1**

| Composition of mouldable substances used in the invention of Examples 1 - 5 stated in % by weight | | | | | |
|---|---|---|---|---|---|
| **Component** | **Example 1** | **Example 2** | **Example 3** | **Example 4** | **Example 5** |
| SEBS | 5 | 5 | 5 | 10 | 8 |
| PIB | 30 | 15 | 15 | 10 | 18 |
| Microcrystalli ne wax | 5 | 5 | 5 | 5 | 5 |
| Petroleum jelly | 10 | | | | |
| Polybutene oil | | 25 | | | |
| Liquid paraffin | | | 25 | 25 | 20 |
| CMC | | | 12 | 20 | 15 |
| Guar Gum | 15 | 20 | | | |
| Pectin | 15 | 10 | 10 | 10 | 8 |
| Gelatine | 18 | 17.5 | 27 | 20 | 25 |
| Zinc white | 2 | 2.5 | 1 | | 3 |

### EXAMPLE 6.

Preparation of a mouldable substance to be used in the invention.

for 30 minutes at 160 °C under a vacuum of 100 mbar and the Hyvis® 2000 was added in four parts to ensure homogeneity during the admixing over a period of 20 minutes. Then, the remains of Hyvis® 2000 was added in four parts at 160 °C over 30 minutes and the vacuum was released. The Hyvis® 10 was added in four parts and mixed for 15 minutes. Wax was added and mixed for 10 minutes.. Then, the heating was turned off, and guar gum and CMC were added at maximum 90 °C under a vacuum of 100 mbar and mixed for 10 minutes. Finally, pectin, gelatine and zinc oxide were admixed at a temperature of 90 °C and mixed for 10 minutes.

The paste was ready to use and may preferably be packed in metered amounts, e.g. in a blister pack or rod. A rod may be rolled and have a release liner on one or both sides. The product is preferably produced and packed under aseptic conditions.

### EXAMPLES 7 - 8

Preparation of mouldable substances to be used in the invention.

In the same manner as described in the Example 2 above, mouldable substances used in the invention were produced having the compositions stated in the below Table 2:

**Table 2**

| Composition of mouldable substances used in the invention of Examples 6 - 8 stated in % by weight | | | |
|---|---|---|---|
| **Component** | **Example 6** | **Example 7** | **Example 8** |
| SEBS (Diblock content about 70%) | 5 | | |
| SIS (Diblock content about 40%) | | 5 | |
| SB (Diblock content about 80%) | | | 5 |
| PIB | | 15 | 15 |
| Polybutene (M_{w} 30.000) | 15 | | |
| Polybutene oil | 25 | 25 | 25 |
| Microcrystalline wax | 5 | 5 | 5 |
| CMC | 10 | 13 | 25 |
| Guar Gum | 15 | | |
| Pectin | 5 | 10 | 8 |
| Gelatine | 18 | 22 | 15 |
| Zinc white | 2 | 5 | 2 |

### EXAMPLES 9 - 10

Preparation of mouldable substances to be used in the invention.

Equal amounts of Kraton® G1726 (SEBS) and Hyvis® 2000 were mixed in a Z Mixer for 30 minutes at 160 °C under a vacuum of 100 mbar and the Hyvis® 2000 was added in four parts to ensure homogeneity during the admixing over a period of 20 minutes. Then, the remains of Hyvis® 2000 was added in four parts at 160 °C over 30 minutes and the vacuum was released. The Hyvis® 10 was added in four parts and mixed for 15 minutes. Resin and wax was added and mixed for 10 minutes each. Then, the heating was turned off, and CMC were added at maximum 90 °C under a vacuum of 100 mbar and mixed for 10 minutes. Finally, pectin, gelatine and zinc oxide were admixed at a temperature of 90 °C and mixed for 10 minutes.

The paste was ready to use and may preferably be packed in metered amounts, e.g. in a blister pack or rod. A rod may be rolled and have a release liner on one or both sides. The product is preferably produced and packed under aseptic conditions.

**Table 3**

| Composition of mouldable substances used in the invention of Examples 9 - 10 stated in % by weight: | | |
|---|---|---|
| **Component** | **Example 9** | **Example 10** |
| SEBS (Diblock content about 70%) | 5 | 5 |
| Polybutene (M_{w} 30.000) | 10 | 5 |
| Polybutene oil | 25 | 25 |
| Resin | 5 | 10 |
| Microcrystalline wax | 5 | 5 |
| CMC | 15 | 15 |
| Pectin | 10 | 10 |
| Gelatine | 24 | 24 |
| Zinc white | 1 | 1 |

## Claims

1. An ostomy appliance comprising
- a body side member (31) having an adhesive wafer (32) for securing said body side member (31) to a user's skin, said body side member (31) having a body side member opening (33) therein for receiving a stoma of said user when said body side member (31) is secured to said user's skin,
- a collection bag (34) having a bag opening (33) therein and means for detachably securing of said collection bag (34) to said body side member (31) with said bag opening (33) in alignment with said body side member opening (33) so as to receive bodily excretions from said stoma,
**characterised in that** said collection bag (34) includes a plastically mouldable adhesive substance (35) at an edge of said bag opening (33) to permit moulding of said plastically mouldable adhesive substance (35) so as to obtain a close fit to said stoma.

2. An ostomy appliance according to claim 1 wherein said collection bag (34) is secured to said body side member (31) by means of an adhesive (36).

3. An ostomy appliance according to claim 1 wherein said collection bag (34) is secured to said body side member (31) by means of mechanical coupling means.

4. An ostomy appliance according to claim 1 wherein said plastically mouldable adhesive substance (35) is cohesive to permit complete removal thereof together with said collection bag (34) from the skin of said user when removing said collection bag (34) from said user's skin.

5. An ostomy appliance according to claim 1 wherein said plastically mouldable adhesive substance (35) is a hardenable substance.

## Patentansprüche

1. Stomaversorgung, umfassend
- ein körperseitiges Element (31) mit einer Klebescheibe (32) zur Befestigung des körperseitigen Elements (31) an der Haut eines Benutzers, wobei das körperseitige Element (31) eine körperseitige Elementöffnung (33) aufweist, um ein Stoma des Benutzers aufzunehmen, wenn das körperseitige Element (31) an der Haut des Benutzers befestigt ist;
- einen Sammelbeutel (34) mit einer Beutelöffnung (33) darin und Mitteln zum lösbaren Befestigen des Sammelbeutels (34) an dem körperseitigen Element (31), wobei die Beutelöffnung (33) mit der körperseitigen Elementöffnung (33) fluchtet, um so Körperexkremente aus dem Stoma aufzunehmen,
**dadurch gekennzeichnet, dass** der Sammelbeutel (34) an einem Rand der Beutelöffnung (33) eine plastisch formbare Klebesubstanz (35) aufweist, um die Formung der plastisch formbaren Klebesubstanz (35) zu ermöglichen, um eine enganliegende Anpassung an das Stoma zu ermöglichen.

2. Stomaversorgung nach Anspruch 1, bei der der Sammelbeutel (34) mittels eines Klebers (36) an dem körperseitigen Element (31) befestigt ist.

3. Stomaversorgung nach Anspruch 1, bei der der Sammelbeutel (34) mittels mechanischer Kopplungsmittel an dem körperseitigen Element (31) befestigt ist.

4. Stomaversorgung nach Anspruch 1, bei der die plastisch formbare Klebesubstanz (35) kohäsiv ist, um deren vollständige Entfernung zusammen mit dem Sammelbeutel (34) von der Haut des Benutzers zu ermöglichen, wenn der Sammelbeutel (34) von der Haut des Benutzers entfernt wird.

5. Stomaversorgung nach Anspruch 1, bei der die plastisch formbare Klebesubstanz (35) eine härtbare Substanz ist.

## Revendications

1. Appareillage stomique comprenant
- un élément latéral de corps (31) possédant une plaquette adhésive (32) destinée à fixer ledit élément latéral de corps (31) à la peau d'un utilisateur, ledit élément latéral de corps (31) possédant une ouverture d'élément latéral de corps (33) destinée à recevoir une stomie dudit utilisateur quand ledit élément latéral de corps (31) est fixé à ladite peau de l'utilisateur,
- un sac collecteur (34) possédant une ouverture de sac (33) et des moyens destinés à une fixation de façon détachable dudit sac collecteur (34) audit élément latéral de corps (31) avec ladite ouverture de sac (33) en alignement avec ladite ouverture d'élément latéral de corps (33) de façon à recevoir des excrétions corporelles provenant de ladite stomie,
**caractérisé en ce que** ledit sac collecteur (34) inclut une substance adhésive plastiquement moulable (35) au niveau d'un bord de ladite ouverture de sac (33) pour permettre un moulage de ladite substance adhésive plastiquement moulable (35) de façon à obtenir un ajustement étroit à ladite stomie.

2. Appareillage stomique selon la revendication 1, dans lequel ledit sac collecteur (34) est fixé audit élément latéral de corps (31) au moyen d'un adhésif (36).

3. Appareillage stomique selon la revendication 1, dans lequel ledit sac collecteur (34) est fixé audit élément latéral de corps (31) par des moyens de couplage mécanique.

4. Appareillage stomique selon la revendication 1, dans lequel ladite substance adhésive plastiquement moulable (35) est cohésive pour permettre son retrait complet conjointement avec ledit sac collecteur (34) de la peau dudit utilisateur lorsque l'on retire ledit sac collecteur (34) de ladite peau de l'utilisateur.

5. Appareillage stomique selon la revendication 1, dans lequel ladite substance adhésive plastiquement moulable (35) est une substance durcissable.
